# FASCICULE DE BREVET EUROPEEN

(11) **EP 0 593 339 B1**
(45) Date de publication et mention de la délivrance du brevet: **28.04.1999**
(21) Numéro de dépôt: 93402469.6
(22) Date de dépôt: 07.10.1993
(51) Int. Cl.: C07K 1/00, A61K 39/29

(54) **Procédé de préparation d'antigènes et de vaccins de l'hépatite A (HAV)**
Verfahren zur Herstellung von Hepatitis Antigenen und Impfstoffen
Process preparation of hepatitis A antigen and vaccine

(30) Priorité: 14.10.1992 FR 9212285
(43) Date de publication de la demande: 20.04.1994
(73) Titulaire: PASTEUR MERIEUX SERUMS ET VACCINS, Société Anonyme :, F-69007 Lyon (FR)
(72) Inventeur: Fanget, Bernard, F-69210 Fleurieux sur l'Arbresle (FR); Françon, Alain, F-69690 Bessenay (FR)
(74) Mandataire: Bernasconi, Jean

(56) Documents cités:
- EP-A- 0 302 692
- EP-A- 0 468 702
- EP-A- 0 522 291
- DATABASE WPI Section Ch, Week 8951, Derwent Publications Ltd., London, GB; Class B04, AN 89-374115 & JP-A-1 279 843 (DENKA SEIKEN KK ET AL.)

## Description

La présente invention a trait à un nouveau procédé de préparation d'antigènes et de vaccins du virus de l'hépatite A (HAV).

La préparation de vaccins efficaces contre l'hépatite A est maintenant connue. Par contre des progrès restent à faire dans le domaine industriel de la culture du virus HAV et de la purification des antigènes de capside de ce picornavirus.

Les procédés initiaux de purification partielle des virions HAV s'avérant trop coûteux ou insatisfaisants pour la production de vaccins, la demande EP-A-0 302 692 a préconisé de soumettre les cellules ayant servi à la multiplication du virus HAV, préalablement lysées, à des opérations d'extraction par solvants organiques associés à des précipitations au polyethylène-glycol (PEG). L'extrait peut être ensuite éventuellement soumis à une chromatographie sur support échangeur d'anions laissant passer les capsides virales, suivie d'une gel-filtration. Ce procédé est présenté comme comportant l'avantage d'éviter toute utilisation de détergents.

Postérieurement la demande EP-A-0 468 702 associe à ce même procédé une deuxième étape de chromatographie sur support échangeur d'anions dans des conditions permettant de retenir les capsides virales qui sont ensuite éluées, l'éluat étant soumis à l'étape de gel-filtration.

Ces procédés présentent l'inconvénient d'être compliqués et de nécessiter l'usage de solvants organiques.

La présente invention se propose de remédier aux inconvénients de l'art antérieur et de fournir un nouveau procédé de préparation d'antigènes et de vaccins de l'hépatite A.

L'invention a pour objet un procédé de préparation d'antigènes et de vaccins de l'hépatite A dans lequel on multiplie le virus HAV sur des cellules compétentes, on lyse, de préférence par sonication, les cellules infectées, on récolte le surnageant et l'on procède à la purification par une opération de chromatographie sur support échangeur d'anions et une opération de gel-filtration, caractérisé en ce que l'on effectue les opérations de purification en présence d'un détergent, de préférence le Tween 80, et en ce que l'opération de chromatographie est effectuée dans des conditions retenant les virions ou capsides virales, qui sont ensuite éluées.

Dans la présente invention la présence du détergent a notamment pour fonction d'éviter les phénomènes d'adsorption des capsides ou virions. Le Tween 80 (Sorbitan mono 9 octadecanoate) s'avère remarquablement efficace, de préférence à des concentrations de 0,001 à 5 %, notamment de 0,1 %. En conséquence l'invention concerne également l'utilisation des détergents, qui, dans cette fonction, sont équivalents au Tween 80.

L'utilisation du détergent, conformément à l'invention, permet d'éviter les étapes d'extraction de l'art antérieur, et de procéder, après simple filtration du surnageant de lyse, à une étape unique de chromatographie à échange d'ions sur un support retenant les capsides virales alors que les contaminants protéiques et nucléiques sont éliminés dans l'effluent. Les capsides antigéniques sont ensuite facilement récupérées par élution.

De préférence le support de chromatographie est constitué par le DEAE Spherodex (Sepracor IBF DEAE Spherodex LS Ref. 61002) mais on peut également utiliser des supports tels que DEAE Sepharose Pharmacia. De préférence la colonne de chromatographie est équilibrée en tampon contenant le détergent, notamment le Tween 80 ; l'éluant peut également contenir le détergent.

On préfère procéder à une concentration de l'éluat après la chromatographie, notamment par ultrafiltration.

L'éluat, de préférence concentré, est ensuite soumis à la gel-filtration, de préférence sur Sepharose 6B CL, en présence du détergent.

Le pic viral obtenu peut alors être à nouveau concentré puis inactivé par un procédé usuel.

D'autres avantages et caractéristiques de l'invention apparaîtront à la lecture de la description suivante, faite à titre d'exemple non limitatif.

### Exemple.

### 1) Culture des cellules.

Les cellules utilisées sont des cellules diploïdes humains de lignée MRC5. La banque de travail est constituée par le 16ème passage.

L'ampoule contenant la banque de travail est décongelée et transférée dans un flacon de culture F 75 cm² dans lequel on introduit du milieu de culture cellulaire en présence de sérum de veau foetal (SVF) et la boîte de culture est placée à l'étuve à 37°C. Lorsque les cellules sont confluentes dans les boîtes de culture, on effectue une opération de trypsination.

On élimine le milieu de culture du récipient de culture, on rince la couche cellulaire avec une solution de PBS et l'on détache les cellules par une solution de trypsine diluée, après quoi on introduit à nouveau du milieu de culture cellulaire, on disperse les cellules dans le milieu et on répartit la suspension cellulaire obtenue dans les nouveaux récipients de culture. On cultive ensuite du 18ème au 38ème passage en utilisant des récipients de plus en plus importants, par exemple en passant d'un flacon de 75 cm² lors du 18ème passage jusqu'au récicipient multitrays CF 40 en utilisant, pour les passages intermédiaires, les récipients de culture usuels correspondant aux volumes croissants à traiter. Après 24 h de culture à 37°C le milieu de culture des récipients CF 40 du 38ème passage est éliminé par aspiration et remplacé par un inoculum constitué de milieu de culture cellulaire contenant du sérum de veau foetal et une quantité de virus Hépatite A de façon à avoir une multiplicité d'infection comprise entre 0,01 et 1. Chaque CF 40 reçoit 8 litres de ce milieu.

Les CF 40 sont alors replacés à l'étuve (température de 35°C) et la propagation virale s'effectue en 21 jours avec les changements de milieu après 8 jours de culture avec du milieu contenant 5 % de sérum de veau, puis après 16 jours avec du milieu ne contenant pas de SVF.

La récolte du virus est effectuée après 21 jours de culture.

### 2) Récolte brute

Le jour de la récolte, les CF 40 sont examinés attentivement et les éventuels CF 40 suspects sont éliminés.

Le liquide de culture surnageant est éliminé.

La couche cellulaire est rincée deux fois à l'aide de deux litres de solution de PBS (solution saline de tampon phosphate).

La couche cellulaire est ensuite rincée par 2 000 ml de solution de trypsine-EDTA (acide éthylènediamine-tétracétique).

Les CF 40 sont replacés à l'étuve 35°C pour 5 minutes.

1 000 ml de solution tampon phosphate 20 mM pH 7,50 sont versés dans chaque CF, les CF sont secoués de façon à décoller toutes les cellules de la surface de culture et la suspension cellulaire est alors récupérée. Les CF sont ensuite rincés par deux fois avec 1 litre de tampon phosphate.

La suspension cellulaire récupérée (volume environ 40 litres pour 12 CF 40) est homogénéisée et traitée aux ultra-sons en continu à une fréquence de 20 KHz dans une cuve à incubation avec un débit compris entre 0,5 et 10 ml/seconde.

Après traitement, la suspension cellulaire est centrifugée à basse vitesse 30 minutes à 2 000 t/mn.

Le surnageant récupéré constitue la récolte brute.

### 3) Addition du détergent

Après prélèvements pour contrôles, une solution de Tween 80 est ajoutée dans la cuve de récolte brute de façon à ajuster la teneur finale en Tween 80 à 0,1 %.

La solution est laissée à + 4°C sous agitation pendant une nuit.

La récolte est alors filtrée à travers des filtres de type Durapore 0,45 et 0,2 µ.

Cette solution constitue donc la récolte filtrée.

### 4) Chromatographie

Le gel DEAE Spherodex est monté en colonne avec HCl 0,1N et préparé par passage de tampon PO₄ 20 mM, pH 7,5 puis solution de formol 2 % NaCl 2 g/l, puis NaCl 1 M.

Pour équilibrer la colonne, puis procéder à la chromatographie, on fait passer du tampon PO₄ 20 mM 7,5 Tween 80 0,1 % jusqu'à équilibrage parfait en pH et osmolalité ; on fait passer l'échantillon dont l'osmolalité ne devra pas être supérieure à 280 mOsm ; on fait passer du tampon PO₄ 20 mM NaCl 0,5 M pH 7,5 Tween 80 0,1 %. Cette opération permet l'élution du virus.

Le débit est de l'ordre de 45 cm/h.

### 5) Concentration

Le matériel utilisé est une cellule d'ultrafiltration type Pellicon équipée de cassettes Sartorius ULTRASART II en ester de cellulose (point de coupure 10 000 daltons).

L'ensemble de l'installation, cassette, module, tuyauterie, est stérilisé à l'aide d'une solution d'eau oxygénée à 5 %.

L'ensemble de l'installation est rincé avec 20 litres de tampon phosphate 20 mM pH = 7,5, Tween 80 0,1 %.

L'éluat de la colonne de DEAE Spherodex, fraction en phosphate 20 mM NaCl 0,5 M Tween 80 0,1 % contenant le virus est concentré.

Le volume final après rinçage des membranes est ajusté à 1 litre.

### 6) Gel-filtration

On utilise une colonne Pharmacia K215/100 remplie avec 26,5 litres de gel Sepharose 6B CL. Hauteur de gel : 73 cm.

L'ensemble de l'installation colonne, tuyauterie, détecteur, est stérilisé par passage de solution de formol 2 %, NaCl 2 %, débit 1,7 l/h.

La colonne est rincée et équilibrée à l'aide de solution de phosphate 20 mM pH = 7,5, Tween 80 0,1 % pendant 48 heures, débit : 2,7 l/h.

Le virus concentré est introduit par gravité dans la colonne ; la purification est effectuée à l'aide de tampon phosphate 20 mM pH = 7,5, Tween 80 0,1 %, débit : 2,7 l/h.

### 7) Concentration finale

Dans des conditions analogues à celles utilisées lors de la concentration précédente, le pic virus issu de la colonne de gel-filtration est concentré. Le volume final après rinçage est ajusté à 2,5 litres. Le virus concentré est filtré à travers une membrane Durapore 0,2 µ.

Ce produit correspond à la récolte purifiée.

### 8) Inactivation

L'inactivation est effectuée par le formol au 1/4 000 pendant 14 j à 37°C.

### 9) Préparation du vaccin

Le vaccin est réalisé à partir de la récolte purifiée inactivée, adsorbée sur hydroxyde d'aluminium, le conditionnement étant effectué selon les méthodes classiques.

## Revendications

1. Procédé de préparation d'antigènes et de vaccins de l'hépatite A dans lequel on multiplie le virus HAV sur des cellules compétentes, on lyse les cellules infectées, on récolte le surnageant et l'on procède à la purification par une opération de chromatographie sur support échangeur d'anions et une opération de gel-filtration, caractérisé en ce que l'on effectue les opérations de purification en présence d'un détergent et en ce que l'opération de chromatographie est effectuée sur un support retenant les virions ou capsides virales qui sont ensuite élués.

2. Procédé selon la revendication 1, caractérisé en ce que l'on utilise comme détergent le Tween 80.

3. Procédé selon l'une des revendications 1 et 2, caractérisé en ce que le détergent est utilisé à des concentrations de 0,001 à 5 %, notamment de 0,1 %.

4. Procédé selon l'une quelconque des revendications 1 à 3, caractérisé en ce que les cellules sont lysées par sonication.

5. Procédé selon l'une quelconque des revendications 1 à 4, caractérisé en ce que l'on effectue la chromatographie sur un support échangeur d'anions dans lequel on fait passer un tampon contenant du détergent, puis on procède à une élution par un éluant contenant également ledit détergent.

6. Procédé selon l'une quelconque des revendications 1 à 5, caractérisé en ce que l'on effectue la gel-filtration sur une colonne équilibrée à l'aide d'un tampon contenant ledit détergent.

7. Procédé selon l'une quelconque des revendications 1 à 6, caractérisé en ce que l'on procède à une concentration de l'éluat contenant le virus issu de la chromatographie, avant la gel-filtration.

8. Procédé selon l'une quelconque des revendications 1 à 7, caractérisé en ce que l'on procède à une concentration finale après la gel-filtration, après quoi la préparation d'antigènes est inactivée.

9. Procédé selon l'une quelconque des revendications 1 à 8, caractérisé en ce que l'on effectue la lyse par sonication à une fréquence de l'ordre de 20 000 Hz.

## Claims

1. Process for the preparation of antigens and vaccines of hepatitis A in which the HAV virus is multiplied on competent cells, the infected cells are lysed, the supernatant is collected and purification is carried out by chromatography on an anion exchange support and a gel-filtration operation, characterised in that the purification operations are carried out in the presence of a detergent and in that the chromatography is carried out on a support which retains the virions or viral capsids which are then eluted.

2. Process according to claim 1, characterised in that Tween 80 is used as the detergent.

3. Process according to either claim 1 or claim 2, characterized in that the detergent is used in concentrations of from 0.001 to 5%, in particular of 0.1%.

4. Process according to any one of claims 1 to 3, characterized in that the cells are lysed by sonication.

5. Process according to any one of claims 1 to 4, characterised in that the chromatography is carried out on an anion exchange support into which is passed a buffer containing detergent before elution is carried out by an eluant also containing said detergent.

6. Process according to any one of claims 1 to 5, characterised in that the gel-filtration is carried out on a column which is equilibrated by means of a buffer containing said detergent.

7. Process according to any one of claims 1 to 6, characterised in that a concentration of the virus-containing eluate produced from the chromatography is carried out before the gel-filtration.

8. Process according to any one of claims 1 to 7, characterised in that a final concentration is carried out after the gel-filtration, after which the antigen preparation is inactivated.

9. Process according to any one of claims 1 to 8, characterised in that the lysis is carried out by sonication at a frequency in the order of 20,000 Hz.

## Patentansprüche

1. Verfahren zur Herstellung von Hepatitis A-Antigenen und -Impfstoffen, in dem der Virus HAV auf erforderlichen Zellen vermehrt wird, die infizierten Zellen aufgelöst werden, der Überstand gesammelt wird und die Reinigung durch ein Chromatographieverfahren auf einem Anionenaustauscherträger und einem Gelfiltrationsverfahren vorgenommen wird, dadurch gekennzeichnet, daß die Reinigungsverfahren in Gegenwart eines Detergens durchgeführt werden und daß das Chromatographieverfahren auf einem Träger durchgeführt wird, der die Virione oder Viruskapside zurückhält, die sofort eluiert werden.

2. Verfahren gemäß Anspruch 1, dadurch gekennzeichnet, daß ein Detergens wie Tween 80 verwendet wird.

3. Verfahren gemäß einem der Ansprüche 1 und 2, dadurch gekennzeichnet, daß das Detergens in Konzentrationen von 0,001 bis 5 % insbesondere von 0,1 % verwendet wird.

4. Verfahren gemäß einem der Ansprüche 1 bis 3, dadurch gekennzeichnet, daß die Zellen durch Sonifikation aufgelöst werden.

5. Verfahren gemäß einem der Ansprüche 1 bis 4, dadurch gekennzeichnet, daß die Chromatographie an einem Anionenaustauscherträger durchgeführt wird, in dem man einen Puffer, der das Detergens enthält, passieren läßt, worauf eine Elution durch ein Elutionsmittel, das ebenfalls das besagte Detergens enthält, vorgenommen wird.

6. Verfahren gemäß einem der Ansprüche 1 bis 5, dadurch gekennzeichnet, daß die Gelfiltration an einer Kolonne durchgeführt wird, die mittels eines Puffers, der das besagte Detergens enthält, äquilibriert ist.

7. Verfahren gemäß einem der Ansprüche 1 bis 6, dadurch gekennzeichnet, daß eine Konzentration des Elutionsmittels, das den aus der Chromatographie hervorgegangenen Virus enthält, vor der Gelfiltration vorgenommen wird.

8. Verfahren gemäß einem der Ansprüche 1 bis 7, dadurch gekennzeichnet, daß eine Endkonzentration nach der Gelfiltration vorgenommen wird, wonach die Herstellung der Antigene inaktiviert wird.

9. Verfahren gemäß einem der Ansprüche 1 bis 8, dadurch gekennzeichnet, daß das Auflösen durch Sonifikation bei einer Frequenz in der Größenordnung von 20 000 Hz durchgeführt wird.
